# EUROPEAN PATENT APPLICATION

(11) **EP 4 745 573 A1**
(43) Date of publication of application: **20.05.2026**
(21) Application number: 24214047.3
(22) Date of filing: 19.11.2024
(51) Int. Cl.: G01N 33/543, B01L 3/00

(54) **IMPROVED PROCESS AND COMPOSITIONS RELATING THERETO**

(71) Applicant: University of Limerick, Limerick V94 T9PX (IE)
(72) Inventor: The designation of the inventor has not yet been filed
(74) Representative: Appleyard Lees IP LLP

(57) **Abstract**

A method of detecting and/or measuring target specific immune cell response to an immune cell target in a sample containing more than one cell, the method comprising the steps of:
(a) providing a sample droplet comprising a portion of the sample, wherein the sample droplet is configured to comprise more than one cell but statistically one or less than one immune cell specific for the target;
(b) contacting an immune cell target with the sample droplet to form an incubation droplet; and
(c) determining the response of a detection agent present in the incubation droplet.

## Description

The present invention relates to a method of detecting and measuring immune cells specific for a particular target in a sample and apparatus for carrying out such a method.

### Background to the Invention

The immune system uses immune cells such as T-cells to protect us against infections and other diseases such as cancer. Circulating in the blood, their main job is to kill cells that they recognise as foreign or abnormal, for example virus-infected cells or cancer cells. The body is able to produce a number of different immune cells and a vast number of different T-cells that each recognise a specific foreign or abnormal molecule (hereafter referred to as the 'target'). The ability of immune cells and T-cells to recognize and kill their targets can be altered in disease, ageing, and following some medical treatments. For example, older people are more vulnerable to COVID-19 because they have fewer T-cells able to recognise and kill virus infected cells.

Inappropriate T-cell responses can occur and are responsible for a range of common medical conditions. These include autoimmune disease, when the immune system attacks healthy tissues of the body, and type IV hypersensitivity when the immune system is activated by otherwise harmless environmental factors.

T-cells play a vital role regulating the immune system, limiting immune responses following infection, reducing allergic responses, and preventing the immune system from attacking normal tissues. Failure of this role can lead to acute and chronic medical conditions such as autoimmune diseases including multiple sclerosis and rheumatoid arthritis.

The immune system can be manipulated for clinical benefit. New T-cell responses can be produced in individuals through vaccination and existing T-cell responses can be modified, an example being immune checkpoint inhibitors that enhance T-cell function in cancer patients. T-cells can be grown outside the body and then used to treat diseases such as cancer and autoimmunity. T-cells can also be modified to alter their properties or their target recognition, such as in CAR-T-cell therapy. Here, T-cells are extracted from the patient, genetically modified to recognise and kill the patient's cancer cells and then returned into the patient. Several CAR-T-cell therapies have been approved by the FDA with many more in development and under review.

The ability to measure how well an immune cell, and in particular a T-cell, can recognize and kill a target (hereafter referred to as "immune cell response" or "T-cell response") would be enormously beneficial. For example, it would allow measurement of the ability of a vaccine specific for a virus to boost the T-cell response to the virus or specific viral protein, nucleic acid or viral particle in vaccinated individuals. It would also allow doctors to determine the effectiveness of CAR-T cells in those treated with this type of therapy. It would aid the diagnosis and management of immune -related conditions such as autoimmunity and allergy. It would also be valuable for researching the function and role in humans and model organisms and advancing the development of immune-based therapies.

Other immune cells, such as natural killer cells, are beginning to be harnessed for the treatment of disease without or with the modifications described above including CAR receptor modification.

Currently, there are four main techniques used to measure T-cell response against specific targets: ELISpot assay, cytokine detection with flow cytometry, ELISA assay, and next generation sequencing. These methods of measuring T-cell response are complex, slow, and laborious to perform (typically 5-10 samples in 24-48 hours, depending on the method used), requiring extensive manual handling steps by skilled staff using specialised equipment and facilities. Data analysis using these methods can be time consuming and subjective. These disadvantages lead to large inter-laboratory variability and a lack of reproducibility. Thus, current assays cannot be implemented at a scale in a high throughput workflow, (e.g. >100 samples per hour). An apparatus and method that allowed a large number of samples and T-cells to be screened for T-cell response would enable the adoption of T-cell response into routine clinical testing (e.g. for the routine monitoring of patients receiving CAR-T cell therapy), or for estimating the success of public health interventions (e.g. vaccination of different demographic and patient groups).

A key problem with developing a robust, high throughput assay is that scientifically or therapeutically meaningful immune responses may be present in the blood at frequencies that are below the limit of detection of existing high throughput assays.

In order to determine whether or a T-cell response is achieved, it is necessary to reliably and consistently discriminate between these low numbers. For example, a strong response is considered to be approximately 100 active T-cells (that is T-cells that respond to the target) out of 1 million total T-cells and a weak T-cell response is considered to be approximately 1 active T-cell out of 1 million total T-cells.

It would also be advantageous to be able to process a large number of samples in a rapid manner.

The present invention provides a significantly improved process and apparatus for determining a T-cell response in a sample which can be used in a variety of applications.

### Summary of the Invention

According to a first aspect of the invention there is provided a method of detecting and/or measuring target specific immune cell response to an immune cell target in a sample containing more than one cell, the method comprising the steps of:
(a) providing a sample droplet comprising a portion of the sample, wherein the sample droplet is configured to comprise more than one cell but statistically one or less than one immune cell specific for the target;
(b) contacting an immune cell target with the sample droplet to form an incubation droplet; and
(c) determining the response of a detection agent present in the incubation droplet.

Step (b) of the method of the first aspect involves contacting an immune cell target with the sample droplet to form an incubation droplet. In some embodiments the immune cell target may be admixed with the sample prior to forming the sample droplet. In such embodiments step (b) is carried out before and during step (a) and involves admixing the immune cell target with the bulk sample prior to forming the sample droplet.

In preferred embodiments step (b) involves admixing an immune cell target with the sample droplet after the sample droplet has been formed. Preferably step (b) is carried out after step (a).

Preferably in step (b) the immune cell target is provided in a target droplet and step (b) involves admixing a target droplet comprising the immune cell target with the sample droplet to form an incubation droplet.

According to a second aspect of the invention there is provided an apparatus for detecting and/or measuring target specific immune cell response to an immune cell target in a sample containing more than one cell, the apparatus comprising:
- a droplet generation system configured to generate one or more target droplets comprising the immune cell target and one or more sample droplets comprising a portion of the sample, wherein the droplet generation system is operable to generate each sample droplet having more than one cell but statistically one or less than one target-specific immune cell per sample droplet;
- a mixing system configured to admix the sample droplet with a target droplet to form an incubation droplet;
- means for determining the presence or absence of response of a detection agent in the incubation droplet.

Preferred features of the first and second aspects of the invention will now be described. In preferred embodiments the method of the first aspect is carried out using the apparatus of the second aspect.

The present invention relates to detecting and/or measuring immune cell response to an immune cell target.

By detecting an immune cell response we mean to refer to determining whether or not a response is achieved.

By measuring an immune cell response we mean to refer to qualitatively assessing the level of immune cell response.

Thus the present invention may be used to provide a qualitative or quantitative assessment of an immune cell response. The invention relates to the response of an immune cell to an immune cell target. The immune cell may be selected from one or more T-cells, NK cells, CAR T-cells, CAR NK cell and mixtures thereof.

In some embodiments the target-specific immune cell may have been modified to recognise the immune cell target for example by infection, vaccination and/or genetic engineering techniques. Such techniques will be known to the person skilled in the art.

The immune cell target may be selected from DNA, RNA, organic molecules, peptides, proteins, metabolites, proteins expressed and/or secreted by pathogens and/or mammalian cells, non-organic molecules, including those that relate to allergic reactions, and mixtures thereof. The selection of an appropriate immune cell target is within the competence of the skilled person.

The present invention relates to determining the immune response in a sample. The sample is suitably provided by a living being. The sample may be provided by a human or animal. Preferably the sample is provided by a human.

Suitably the sample comprises bodily fluid and/or tissue. The sample may be one that has been previously removed from a subject. In a further related embodiment, the bodily fluid and/or tissue is taken from a subject. The subject may have been previously vaccinated to recognise and/or respond to the immune cell target, and/or the subject has been infected by a pathogen which expresses said immune cell target, and/or the subject has undergone immunotherapy so that the immune cell from the subject recognises and/or responds to the immune cell target.

In the present invention one or more sample droplets are generated. Each sample droplet comprises a portion of the sample having more than one cell but statistically one or less than one target-specific immune cell per sample droplet.

The sample comprises more than one cell. It may comprise multiple cells of the same type. It may comprise multiple types of cell. It may comprise multiple cells of multiple types.

Each sample droplet statistically comprises one or less than one target-specific immune cell. However various other types of cell may be present.

For example, if the sample is a blood sample it may comprise white blood cells, red blood cells, platelets and various immune cells.

However in the present invention the sample droplet size and sample concentration are selected such that on average each sample droplet will comprise one or less than one target-specific immune cell. This means that for each sample droplet a binary determination can be made as to the presence or absence of the immune cell target. Thus the target-specific immune cells are suitably arranged in a Poisson or bionomial distribution.

The droplet generation system is suitably a microfluidic system. Suitable the droplets are provided by a dipping head connected to a pump. Suitably such configurations will be known to the person skilled in the art.

The tube diameter of the apparatus will suitably be selected to generate droplets of the desired size.

The size of the sample droplet is suitably selected to ensure a suitable microenvironment to maintain cell health.

In preferred embodiments the sample droplets have a volume of from 500nL to 1 mL.

Preferably the target droplets have a volume of from 500nL to 1 mL.

In step (b) of the method of the present invention an immune cell target is admixed with the sample droplet.

Preferably the immune cell target is provided in a target droplet.

The apparatus of the present invention includes a droplet generation system which provides the one or more target droplets and the one of more sample droplets.

Preferably the apparatus is configurated to generate sample droplets and target droplets in a pattern which facilitates mixing.

The sample droplet is mixed with the target droplet.

In some embodiments the target droplet and the sample droplet may be introduced to the mixing system via different feeds. In preferred embodiments the sample droplet and the target droplet are provided by the same feed into the mixing system. They may be separated by an inert material, for example a biocompatible oil.

In some embodiments the mixing system is part of the microfluidic system and comprises a mixing chamber.

The mixing chamber is suitably a wide portion of tube in which sequential droplets can coalesce.

In preferred embodiments in which sample droplets and target droplets are alternatively provided the mixing system includes wider sections of microfluidic tube in which two sequential droplets can coalesce.

Admixture of the sample droplet and the target droplet provides an incubation droplet.

The incubation droplet further comprises a detection agent.

The detection agent may be provided separately to the target droplet and the sample droplet as it may be provided within the sample droplet and/or the target droplet.

Preferably the detection agent is provided in the sample droplet.

In some embodiments the apparatus may further comprise means for admixing the detection agent with the sample.

In some embodiments the method may include a step of mixing the sample with the detection agent.

By detection agent we mean to refer to any material or combination of materials which provides an indication of the presence of a marker of target specific immune cell activation. It will be apparent to the skilled person that there are many different markers that are indicative of immune cell activation. For example, the marker of target specific immune cell activation may be expressed and/or secreted by the target specific immune cell. Alternatively, the marker of target specific immune cell activation may be secreted or expressed by a different immune cell present in the sample. The marker is a molecule which is generated when the target specific immune cell is activated. Markers of immune cell activation are well known. The choice of detection agent will depend on the marker and the immune cell interaction being monitored.

Suitable markers will be known to the person skilled in the art and include, for example increased levels of cell surface proteins such as CD25, CD69, OX40, 4-1BB and increased levels of cytokines such as Interferon gamma (IFN-g), Tumour Necrosis Factor alpha (TNFa), Interleukin-2 (IL2), Interleukin 4 (IL4), Interleukin-10 (IL10), Interleukin-17 (IL17), Macrophage Inflammatory Protein 1 (MIP1).

In preferred embodiments the marker is Interferon gamma (IFN-y).

In some embodiments the detection agent may comprise one or more antibodies specific for the marker of target-specific immune cell activation. The term "antibody" as used herein broadly refers to any immunoglobulin (Ig) molecule, or antigen binding portion thereof, comprised of four polypeptide chains, two heavy (H) chains and two light (L) chains, or any functional fragment, mutant, variant, or derivation thereof, which retains the essential epitope binding features of an Ig molecule. In a full-length antibody, each heavy chain is comprised of a heavy chain variable region or domain (abbreviated herein as HCVR) and a heavy chain constant region. The heavy chain constant region is comprised of three domains, CH1, CH2 and CH3. Each light chain has a light chain variable region or domain (abbreviated herein as LCVR) and a light chain constant region. The light chain constant region is comprised of one domain, CL. The heavy chain and light chain variable regions can be further subdivided into regions of hypervariability, termed complementarity determining regions (CDR), interspersed with regions that are more conserved, termed framework regions (FR). Each heavy chain and light chain variable region are composed of three CDRs and four FRs, arranged from amino-terminus to carboxy-terminus in the following order: FR1, CDR1, FR2, CDR2, FR3, CDR3, FR4. Immunoglobulin molecules can be of any type (e.g., IgG, IgE, IgM, IgD, IgA and IgY), class (e.g., IgG 1, IgG2, IgG 3, IgG4, IgAl and IgA2) or subclass.

In some embodiments the antibody may be human. However in some preferred embodiments non-human animal antibodies are used. Suitable antibodies will be known to the person skilled in the art and include mouse, rabbit, rat and camelid.

Antibody mimetics, for example aptamers may also be used herein as detection agents.

An antibody fragment as used herein is a portion of an antibody, for example as F(ab')2, Fab, Fv, scFv, heavy chain, light chain, heavy (VH), variable light (VL) chain domain and the like, that binds to the target antigen. Functional fragments of a full length antibody, i.e. antigen binding fragments of an antibody, retain the target specificity of a full antibody. Recombinant functional antibody fragments, such as Fab (fragment antigen binding region), scFv (single chain variable chain fragments) and single domain antibodies (dAbs) have therefore been used to develop therapeutics as an alternative to therapeutics based on full monoclonal antibodies (mAbs). scFv fragments (~25kDa) consist of the two variable domains, VH and VL. Naturally, VH and VL domain are non-covalently associated via hydrophobic interaction and tend to dissociate. However, stable fragments can be engineered by linking the domains with a hydrophilic flexible linker to create a single chain Fv (scFv). The smallest antigen binding fragment is the single variable fragment, namely the single variable heavy (VH) or single variable light (VL) chain domain. VH and VL domains respectively are capable of binding to an antigen. Binding to a light chain/heavy chain partner respectively or indeed the presence of other parts of the full antibody is not required for target binding. The antigen-binding entity of an antibody, reduced in size to one single domain (corresponding to the VH or VL domain), is generally referred to as a "single domain antibody" or "single immunoglobulin variable domain". A single domain antibody (-12 to 15 kDa) thus consists of either the VH or VL domain, but it does not comprise other parts of a full length antibody. Single domain antibodies derived from camelid heavy chain only antibodies that are naturally devoid of light chains as well as single domain antibodies that have a human heavy chain domain have been described (Muyldermans J Biotechnol. 2001 Jun;74(4):277-302; Holliger Nat Biotechnol. 2005 Sep;23(9):1126-362). Antigen binding single VH domains have also been identified from, for example, a library of murine VH genes amplified from genomic DNA from the spleens of immunized mice and expressed in E. coli (Ward et al., 1989, Nature 341: 544-546). Ward et al. named the isolated single VH domains "dAbs" for "domain antibodies." The term "dAb" or "sdAb" as used herein generally refers to a single immunoglobulin variable domain (VH, VHH or VL) polypeptide that specifically binds antigen. Such a molecule only has the VH or VL binding domain respectively, but does not comprise other parts of a full length antibody. Unless otherwise specified, as used herein, the term refers to a single domain antibody that has a VH domain. The terms "single domain antibody", "sdAb", "VH domain antibody", "single VH domain antibody", "VH single domain antibody", "single variable domain", "single variable domain antibody", "single variable heavy chain domain antibody" or immunoglobulin single variable domain (ISV)" are thus all well known in the art and describe the single variable fragment of an antibody that binds to a target antigen. These terms are used interchangeably herein. These terms above and specifically "single heavy chain domain antibody", "single variable heavy chain domain antibody" "single VH domain antibody", "single VH domain antibody", "ISV", and "VH single domain" as used interchangeably herein describe a part of an antibody, i.e. the single heavy chain variable fragment of an antibody, e.g. the VH domain, which retains binding specificity to the antigen in the absence of light chain or other antibody fragments. Such a molecule, e.g. a single variable heavy chain domain antibody is capable of binding to an antigen in the absence of light chain. A single variable heavy chain domain antibody does not comprise other parts of a full length antibody; it only includes the VH domain. Thus, as used herein, these terms specify a binding moiety that is solely made up of the VH domain and does not have other parts of an antibody, that is it does not have a light chain or part thereof and does not have a constant region or part thereof.

The detection agent used in the present invention typically includes an antibody species which interacts with the marker produced when the target immune cell is activated.

By antibody species we mean to include single antibodies, portions of antibodies, combinations of antibodies, antibody mimetics and mixtures thereof.

In some embodiments the antibody may be directly conjugated to a detectable moiety for example a fluorescent species. In some embodiments the detection agent may include a first antibody which interacts with the marker and one or more further components which interact to provide a detectable signal.

For example, a first antibody may interact with a second antibody that is conjugated to a fluorescent species, altering the fluorescent properties of the species.

Various systems of this type are known and are commonly used in the art to determine if molecules are within close proximity, for example time-resolved fluorescence resonance energy transfer (TR-FRET).

When the detection agent comprises multiple components different parts of the detection agent may be added separately. For example one component of the detection agent may be provided in the sample droplet and one or more further components may be provided in a separate detection droplet.

In some embodiments such a detection droplet may added to the same feed as the sample droplet and the target droplet. In some embodiments the detection droplet may be separately added.

Alternatively, an enzyme may be split and different parts joined to the antibodies rendering it inactive until the antibodies interact with the marker, brining the two parts into close proximity and restoring enzymatic activity that can be detected using a suitable substrate.

Suitable enzymes will be known to the person skilled in the art and include, for example, Beta-galactosidase and luciferase.

In one preferred embodiment the detection agent may comprise a first antibody conjugated to one half of a luciferase enzyme and a second antibody conjugated to the corresponding half of the enzyme. The whole enzyme agent may a polypeptide/protein which catalyses a reaction that forms a luminescent product when exposed to a substrate. One example of such technology is commonly known as Lumit^{®} technology which comprises two distinct antibodies which each bind to the same marker. The two antibodies routinely bind to antigen binding site locations on the marker that are near to each other so that when the two antibodies bind to the marker the two halves of the enzyme brought into contact with each other. Each antibody may comprise one half of a enzymatic agent The term "half" in this instance refers to two parts of the enzymatic agent. The halves of the enzymatic agent may be different sizes in terms of weight, 3D space or number of chemical entities. The enzymatic agent may be a NanoBiT^{®} enzyme which is formed of an LgBiT half and a corresponding SmBiT half. The first antibody may be conjugated to either the LgBiT or the SmBiT half and the second antibody conjugated to the corresponding half. The skilled person will understand that other detection agents may be used which follow the same principles as the Lumit^{®} system detailed above.

The admixture of the target droplet and the sample droplet provides an incubation droplet. Suitably the method of the present invention includes a step between step (b) and step (c) of incubating the incubation droplet.

The apparatus is suitably configured to incubate the incubation droplet. Incubation is suitably carried out for a period of time sufficient to allow the target specific immune cell to interact with the immune cell target under conditions that support the viability of the cells present in the sample droplet.

Selection of suitable incubation conditions will be within the competence of the skilled person.

The present invention relates to the detection and/or measurement of immune cell response. The invention is particularly useful in the detection and/or measurement of a T cell response.

The T-cell target may be selected from DNA, RNA, organic molecules,, peptides or proteins corresponding to metabolites or proteins produced by micro-organisms or mammalian cells and non-organic molecules related to allergy.

The T-cell response is suitably measured by detecting functional changes in the target-specific T-cell. An example is calcium flux, which occurs rapidly following activation of the target-specific T-cell. Changes in the level of RNA or protein contained in the target-specific T-cell or secreted into the droplet can also be measured, an example being production of a cytokine, for example IFN-γ.

Certain changes in function, RNA or protein occur universally in T-cells following activation and can therefore measure the total T-cell response to a target regardless of the role of the T-cell in the immune system. An example of such changes are calcium flux measured by a calcium-sensitive dye, or alterations in levels of the RNA or protein encoded by the NR4A3 gene. Suitable calcium sensitive dyes include Fura-2 and Indo-1.

Other changes in function, RNA or protein can be used to determine the function of the target-specific T-cells. An example of such a functional change is alteration of fluorescent probes containing cleavage sites for cytotoxic effector molecules such as granzymes used by T-cells to kill target cells, thereby detecting T-cell cytotoxicity. Examples of changes in RNA or protein that can be used to identify function include Interferon gamma (IFN-g), Tumour Necrosis Factor alpha (TNFa), Interleukin-2 (IL2), Interleukin 4 (IL4), Interleukin-10 (IL10), Interleukin-17 (IL17), Macrophage Inflammatory Protein 1 (MIP1). The cytokines may be produced by CD8 and CD4 T-cells, and/or produced by regulatory T-cells.

An activated target-specific T-cell within the droplet can produce messenger molecules such as cytokines that alter the functional properties or levels of RNA or levels of protein of bystander cells present in the droplet. These changes in bystander cells can also be measured by the above methods to identify the presence of a target-specific T-cell in the droplet and may amplify the signal produced by a single target-specific T-cell. Other changes in bystander cells that can be measured include interferon response genes.

Changes in the expression of RNA or levels of protein can be measured by a nucleic acid based amplification reaction and/or fluorescent dye and/or antibody system and/or fluorescent probe. Examples of methods of RNA detection include reverse transcription polymerase chain reaction (RT-PCR), reverse transcription loop-mediated isothermal amplification (RT-LAMP), and nucleic acid sequence based amplification and transcription mediated amplification (NASBA).

The present invention may involve a qualitative or quantitative determination of an immune cell response, for example a T cell response.

Step (c) of the method of the first aspect of the present invention involves determining the response of a detection agent present in the incubation droplet.

The apparatus of the second aspect of the invention includes means for determining the presence or absence of response of a detection agent in the incubation droplet.

The response may be determined qualitatively or quantitatively.

A qualitative assessment may simply involve observing the presence or absence of a signal from the detection agent in an incubation droplet. This allows a conclusion to be inferred regarding the presence or absence of a target specific immune cell response.

In some preferred embodiments the present invention involves the provision of multiple sample droplets which are admixed with multiple target droplets to provide multiple incubation droplets.

By counting the number of droplets which have a positive or negative response to the detection agent, a quantitative assessment of the target specific immune cell response can be determined.

The present invention offers significant advantages compared with methods of measuring T-cell response of the prior art. In particular the invention avoids the need for complex washing steps and may be used as a continuous process allowing multiple samples to be assessed quickly.

The present invention finds utility in a wide variety of applications. It may be used in therapeutic, diagnostic applications; it can be used to provide a prognosis for patients and assess treatment efficacy; the invention may be useful in gene therapy and vaccination applications, and it can be used to study and research the immune system in humans and other organisms.

The invention will now be further described with reference to the accompanying figures and the examples which follow.

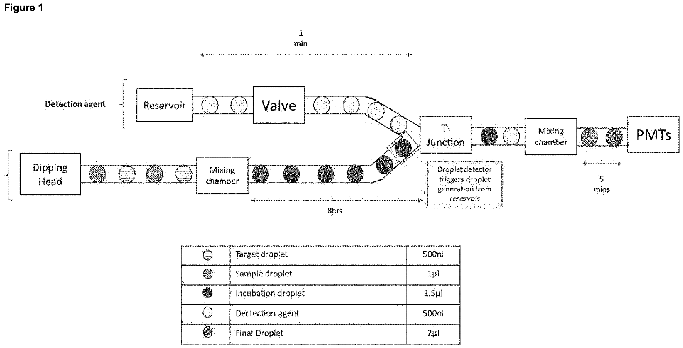

Figure 1 is a schematic diagram representing the major components of the apparatus.

The dipping head alternates between the sample and a composition comprising the immune cell target. In some embodiments a portion of the detection agent may be mixed with the sample. The tube diameter is 0.75 mm and the mixing chamber or drop off junction is a portion of tube having a diameter of 1.5 mm. The flow rate is 12 ml/min. There is a droplet detector at the T-junction to indicate when a droplet of detection agent needs to be delivered.

PMT is a photomultiplier tube.

Figure 2 is a graph that shows the output voltage overtime when artificially positive and negative droplets are passed through the detection portion of the apparatus in series. Output voltage is a direct measure of fluorescence of each droplet.

Figure 3 is a graph showing the output voltage over time when artificially positive and negative droplets are passed through the detection portion of the apparatus in series. 10X PBS was added to the substrate buffer in both the positive and negative droplets. Output voltage is a direct measure of fluorescence of each droplet.

Figure 4 is a graph showing the output voltage over time when droplets having various concentrations of cytokine (IFN-γ) are run through the apparatus in series. Output voltage is a direct measure of fluorescence of each droplet. Underneath the graph is a schematic diagram showing the concentration of cytokine present in each droplet run through the apparatus in series.

Figure 5 is a graph showing the dissolution curve generated using the data from Figure 4. The limit of detection (LOD) is calculated from this curve as approximately 0.15 pg of cytokine.

Figure 6 is a graph showing the output voltage over time when droplets formed of supernatant containing IFN-γ produced by secretion from SNT-13 cells and negative droplets are run through the apparatus in series. Output voltage is a direct measure of fluorescence of each droplet.

Figure 7 is a graph showing the output voltage over time when negative droplets (Jurkat cells) and positive droplets (SNT-13 cells & Jurkat cells) were passed through the apparatus in sequence. Output voltage is a direct measure of fluorescence of each droplet. Positive and negative droplets are marked with a star where these were detectable above background noise (negative droplets were not always detectable above background).

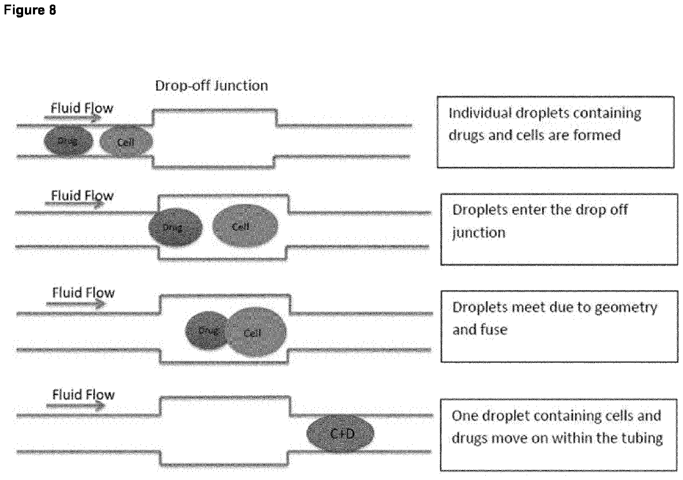

Figure 8 is a schematic diagram showing the arrangement of a drop off junction or mixing chamber which is incorporated into the apparatus when two droplets require mixing together.

The invention will now be further described with reference to the following non-limiting examples.

### Example 1

### Initial Device and Method Testing

Initially the method and device were tested to confirm it was possible to segment a sample into droplets and then to differentiate those droplets containing a concentration of cytokine with those droplets lacking the required concentration of cytokine.

### Method

Droplets comprising Lumit Antibodies, IFN-γ and substrate (Lumit Buffer B) were formed using the device. Negative (0 pg) and positive (20 pg) droplets were passed through the device. This was carried out to mimic the situation that would occur when a sample containing both T-cells specific for the target and non-specific T-cells was passed through the device in the form of droplets.

This example was carried out using relatively high IFN-γ levels (20 pg/droplet) as a test of the instrument and chemistry.

### Results

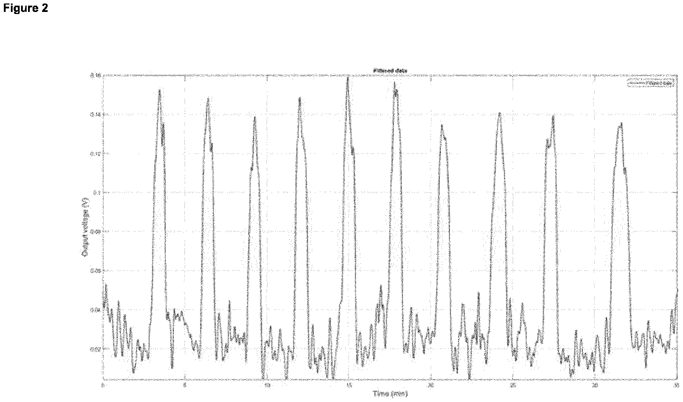

The results of this example are shown in Figure 2. The positive droplets containing 20 pg/droplet of IFN-γ are clearly visible (∼0.15V) as separate peak on the graph. The positive peaks are separated from each other by negative droplets containing 0 pg/droplet of IFN-γ and are indistinguishable from background noise.

### Example 2

### Effect of Salt Concentration on Signal Intensity

The aim of this example was to assess how salt in the form of phosphate buffered saline (PBS) affects the signal intensity of each sample droplet and to determine whether the signal-to-noise ratio is sufficient to maintain a clear distinction between positive droplets (droplets containing T-cells specific for the target) and negative droplets.

### Method

Droplets comprising Lumit Antibodies, IFN-γ and substrate (Lumit Buffer B) including 10X PBS were formed and incubated using the device. Negative (0 pg) and positive (0.25 pg) droplets were passed through the device in sequence.

### Results

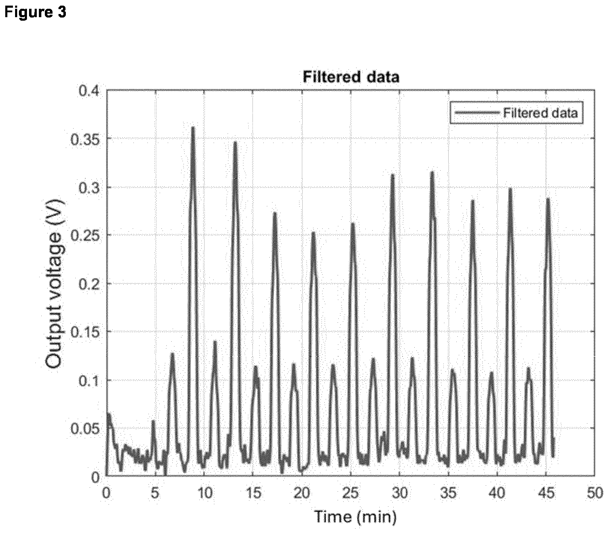

As shown in Figure 3 an increase in signal intensity of the positive droplets was observed with the addition of 10X PBS. Despite the increase in signal intensity this did not compromise the signal-to-noise ratio. Positive droplets containing 0.25 pg of IFN-γ cytokine were clearly distinguishable from negative droplets. There was a notable increase in measured signal for positive droplets (~0.3V). In addition, the negative droplets are also distinguishable from the background noise (~0.1V).

### Example 3

### Quantification of T-cellresponse

This example was carried out to determine whether it is possible to quantify T-cell responses so that T-cells specific for the target that produce low levels of cytokine can be assessed. The aim was also to investigate whether it is possible for the response of T-cell specific for the target to be quantifiably measured and what the limit of detection (LOD) is for the apparatus and method.

### Method

Droplets of IFN-γ (0.25 pg/droplet - 2 pg/droplet), Lumit antibodies and substrate with 10X PBS were formed and incubated using the device. Subsequently the signal of each droplet was measured using the device. The first 20 droplets were negative (0 pg) and positive (0.25 pg) in sequence. Followed by 2 negative and two positive of 0.5 pg, 1 pg and 2 pg.

### Results

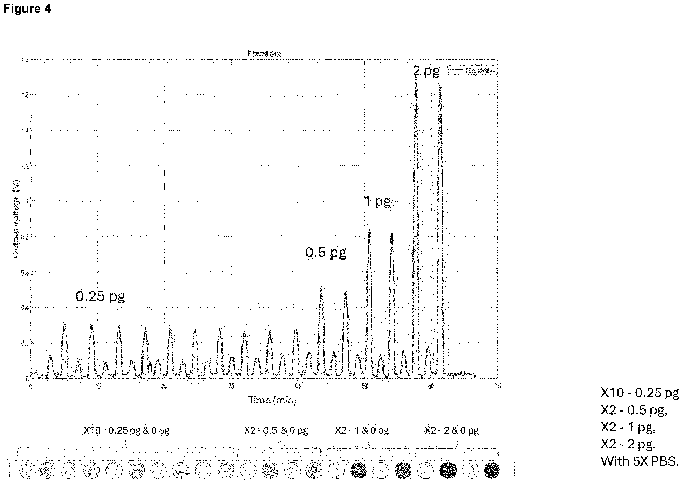

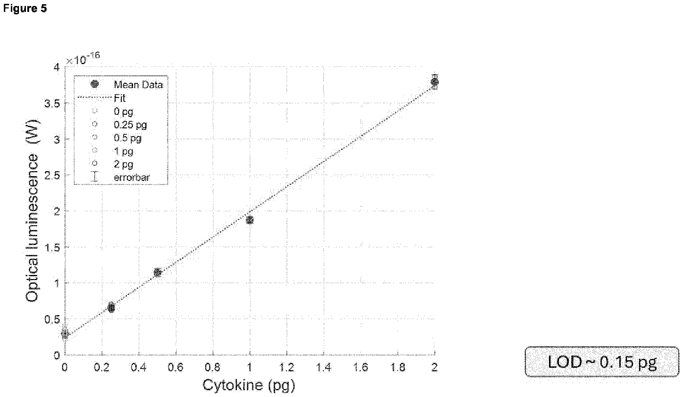

As shown in Figure 4 there was a clear increase in signal corresponding to higher concentrations of IFN-γ. The data presented in Figure 4 clearly demonstrates that the quantification of cytokine release from T-cells is possible using the apparatus if required. The data contained in Figure 4 was reanalysed to produce the dissolution curve shown in Figure 5. From the curve of Figure 5 it was possible to determine that the limit of detection (LOD) is approximately 0.15 pg of protein.

### Example 4

### Proof of Concept using SNT-13 cells

Following the demonstration that the apparatus is capable of determining whether a sample is artificially positive or negative using IFN-γ the next step was to demonstrate that the apparatus and method can be used with supernatant containing IFN-γ produced by secretion from cells.

### Method

SNT-13 cells were incubated overnight. Supernatants were then collected and combined with Lumit antibodies and substrate buffer. The negative (0 pg) and positive (~0.25 pg) droplets are shown in sequence. The SNT-13 cell line was established from patients with CAEBV and is an EBV-positive γδ T-cell line.

### Results

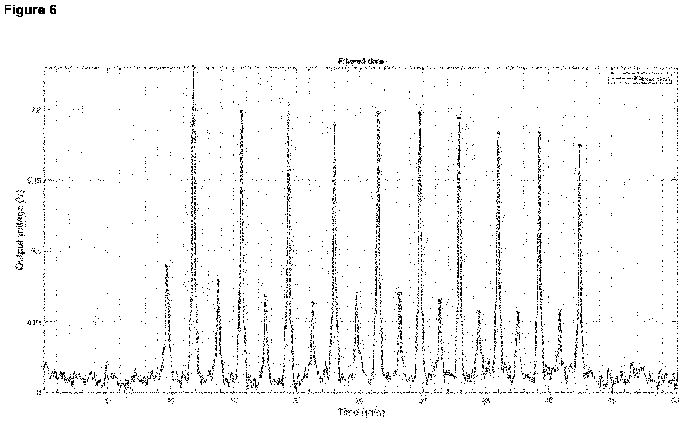

The results of this example are shown in Figure 6. As shown in Figure 6, a clear signal comparable to recombinant human IFN-γ produced in the previous examples was detected. This demonstrates that the apparatus and method are suitable for detecting IFN-γ produced naturally by T-cells and is sensitive enough to detect a natural lower level of IFN-γ. The SNT-13 cell line is also suitable for use in the method and apparatus.

### Example 5

### Full Proof of Concept Using SNT-13 and Jurkat Cells Incubated and Assessed Using the Apparatus

Following on from Example 4, Example 5 was designed to determine whether the apparatus and method was suitable to detect IFN-γ secreted from cells incubated and screened using the apparatus itself.

### Method

A single population of Jurkat cells and a mixed population of SNT-13 cells and Jurkat cells were incubated with Lumit antibodies and droplets formed from these populations using the apparatus. The Jurkat cell line is a standard cell line that does not secrete IFN-γ. Negative droplets (Jurkat) and positive droplets (SNT-13 & Jurkat) were passed through the apparatus in sequence.

### Results

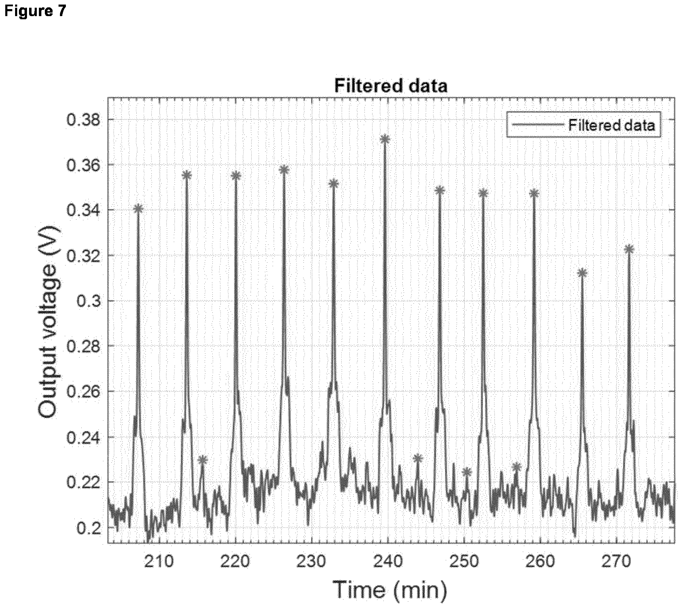

The data shown in Figure 7 demonstrates that there was a detectable signal in the positive droplets (SNT-13 and Jurkat). The signal corresponding to the negative droplets (Jurkat) was no higher than background sample noise. This data demonstrates that the apparatus and method can be used to incubate cells in droplets and to differentiate between droplets containing T-cells specific for the target and droplets that do not contain active T-cells specific for the target.

## Claims

1. According to a first aspect of the invention there is provided a method of detecting and/or measuring target specific immune cell response to an immune cell target in a sample containing more than one cell, the method comprising the steps of:
(a) providing a sample droplet comprising a portion of the sample, wherein the sample droplet is configured to comprise more than one cell but statistically one or less than one immune cell specific for the target;
(b) contacting an immune cell target with the sample droplet to form an incubation droplet; and
(c) determining the response of a detection agent present in the incubation droplet.

2. According to a second aspect of the invention there is provided an apparatus for detecting and/or measuring target specific immune cell response to an immune cell target in a sample containing more than one cell, the apparatus comprising:
- a droplet generation system configured to generate one or more target droplets comprising the immune cell target and one or more sample droplets comprising a portion of the sample, wherein the droplet generation system is operable to generate each sample droplet having more than one cell but statistically one or less than one target-specific immune cell per sample droplet;
- a mixing system configured to admix the sample droplet with a target droplet to form an incubation droplet;
- means for determining the presence or absence of response of a detection agent in the incubation droplet.

3. A method or apparatus according to claim 1 or claim 2 wherein the target-specific immune cell is selected one or more of T-cells, NK cells, CAR T-cells, CAR NK cells and mixtures thereof.

4. A method or apparatus according to any preceding claim, wherein the target-specific immune cell has been modified to recognise the immune cell target, for example by infection, vaccination and/or genetic engineering techniques.

5. A method or apparatus according to any preceding claim, wherein the immune cell target is selected from DNA, RNA, organic molecules, peptides, proteins, metabolites, proteins expressed and/or secreted by pathogens and/or mammalian cells, non-organic molecules, including those that relate to allergic reactions, and mixtures thereof.

6. A method or apparatus according to any preceding claim, wherein the sample is bodily fluid and/or tissue provided by a human subject.

7. A method or apparatus according to claim 6 wherein the sample has been previously removed from the subject.

8. A method or apparatus according to claim 6 or claim 7 wherein the subject has been previously vaccinated to recognise and/or respond to the immune cell target, and/or the subject has been infected by a pathogen which expresses said immune cell target, and/or the patient has undergone immunotherapy so that the immune cell from the subject recognises and/or responds to the immune cell target.

9. A method or apparatus according to any preceding claim wherein the detection agent comprises a material or combination of materials which provides an indication of the presence of a marker of target specific immune cell activation.

10. A method or apparatus according to any preceding claim, wherein the detection agent comprises one or more antibodies specific for a marker of target-specific immune cell activation.

11. A method or apparatus according to claim 10, wherein the one or more antibodies comprises a first antibody conjugated to one half of an enzyme and a second antibody conjugated to the corresponding half of the enzyme..

12. An apparatus according to any of claims 2 to 11 wherein the apparatus is a microfluidic system.

13. An apparatus according to any of claims 2 to 12 wherein the droplet generation system is configured to generate a series of droplets which alternate between sample droplet and target droplet.

14. An apparatus according to any of claims 2 to 13 wherein the mixing system comprises a wide portion of tube in which sequential droplets can coalesce.

15. A method or apparatus according to any preceding claim wherein each droplet has a volume of between 500 nL and 1 mL.
